# EUROPEAN PATENT APPLICATION

(11) **EP 4 279 122 A1**
(43) Date of publication of application: **22.11.2023**
(21) Application number: 22174484.0
(22) Date of filing: 20.05.2022
(51) Int. Cl.: A61N 1/365, A61N 1/368, A61N 1/375

(54) **PACEMAKER AND OPERATION METHOD OF SUCH PACEMAKER**

(71) Applicant: BIOTRONIK SE & Co. KG, 12359 Berlin (DE)
(72) Inventor: Swenson, Kurt, Dayton, 97114 (US); Miller, David, Lake Oswego, 97035 (US); Freiberg, Karl-Heinz, Woodburn, 97071 (US); McMillan, Brad, Lake Oswego, 97035 (US); Young, Daniel, Portland, 97202 (US)
(74) Representative: Biotronik Corporate Services SE

(57) **Abstract**

The invention is generally directed to a cardiac pacemaker (10) for a patient's heart (20), for example an ILP which realizes integrated circuit space conservation and simple design that covers many modes of operation even though robust behaviour requires complex dynamic adaptive algorithms. The pacemaker comprises a processing unit (120), a detector (126) and a pacing signal generator (124), wherein the processing unit, the detector and the pacing signal generator are electrically interconnected, wherein the detector is configured to detect electrical signals of the heart, for example an intracardiac electrogram (IEGM), and to transmit these signals to the processing unit (120), wherein the processing unit is configured to perceive an intrinsic ventricular signal and an intrinsic atrial signal from the signals received from the detector, to enable or disable the perception of the intrinsic atrial signal, to produce a ventricular pacing control signal (Vp).

## Description

The invention is generally directed to a cardiac pacemaker and an operation method of such pacemaker, a respective computer program product and computer readable data carrier.

A cardiac pacemaker (or artificial pacemaker) is a medical device that generates electrical pulses delivered by electrodes connected to or fixed at the pacemaker to cause the heart muscle chambers (i.e. the atria and/or the ventricles) to contract and therefore pump blood. By doing so this device replaces and/or regulates the function of the electrical conduction system of the heart. One purpose of a pacemaker is to maintain an adequate heart rate (cardiac rate), either because the heart's natural pacemaker is not fast enough, or because there is a block in the heart's electrical conduction system. Additionally, or alternatively, the pacemaker may stimulate different positions within the ventricles to improve their synchronization of the ventricles or provide defibrillation functions in order to treat life-threatening arrhythmias. Modern pacemakers are externally programmable and allow a health care provider (HCP) to select the optimal pacing mode(s) for individual patients.

A conventional pacemaker comprises a controlling and generator device comprising a processing unit and a power source external of the patient's heart and electrodes that are implanted within the heart's muscle. The electrodes are connected via leads and a header located at the device to the device. In most cases the device is implanted transcutaneous in the front of the chest in the region of the left or right shoulder. An implantable intracardiac pacemaker (also known as implantable leadless pacemaker - ILP) is a miniaturized pacemaker which is entirely implanted into the ventricle (V) or atrium (A) of a patient's heart. ILPs are considered to be crucial to the future of cardiac pacing. Alternative or additional functions of conventional or intracardiac pacemakers comprise providing other electrical or electromagnetic signals to the heart or its surrounding tissue and sensing electrical or electromagnetic signals (e.g. signals from electrical depolarization fields), physical motion signals, or other physiological parameters of the heart and/or its surrounding tissue such as the intrinsic (i.e. the heart's natural) atrial contraction or the intrinsic (i.e. the heart's natural) ventricular contraction. Due to the highly restricted device size, an ILP has a small battery capacity.

The pacing functionality of a conventional or ILP is aimed at staying synchronized as far as possible with the heart's natural activity.

An ILP may be operated in VDD pacing mode (i.e. a pacing mode in which the ventricle is stimulated according to the intrinsic atrial signal and AV conduction monitoring). In the VDD mode, the pacemaker synchronizes ventricular pacing with the intrinsic atrial timing by sensing when atrial depolarizations or contractions (i.e. the intrinsic atrial signals) occur. In an ILP that is implanted in the right ventricle, the atrial contraction information can be detected as a far field signal, which may have lower reliability or accuracy than in a dual chamber conventional pacemaker where there is a lead the in right ventricle as well as in the right atrium.

Additionally, pacemakers are known to use a specific rate for determining the rate at which to pace the heart. The specific rate may be realized by counting a clock signal of an interval corresponding to the specific rate. The simplest pacemakers use a fixed, typically programmable, rate which meets the needs of the patient under most circumstances. Using a sensor derived rate that is based on a demand correlated measurement, such as acceleration, is common. For replacing failed AV conduction signals, AV synchronous pacemakers attempt to pace the ventricle at a rate that corresponds with a detected atrial signal (e.g. atrial contraction signal or atrial depolarization signal). It is also possible to deduce a physiologically missing electrical heart rate signal from other systems in the body (e.g. the brain and baroreceptors) from other sensors.

An inherent rate used by all modern pacemakers is the intrinsic rate of the heart. When the intrinsic rate is near the pacing rate, the refractory periods of the cardiac conduction paths tend to block the conduction of the intrinsic electrical signals, but when the intrinsic signal is at a rate that is significantly faster than the pacing rate, the signals may conduct and trigger heart chamber contractions. These are sensed by the pacemaker and such sense detections are used to inhibit pacing.

Abrupt large changes in the cardiac rate may trigger undesirable and potentially dangerous arrhythmias and may be noticeable and uncomfortable to some patients. Accordingly, such large changes in cardiac rate should be avoided.

Accordingly, there is the need for a cardiac pacemaker which copes with intrinsic rate changes and failed detection of intrinsic atrial signals as well as avoids large changes in the pacing rate. Additionally, in case of an ILP, the pacemaker must meet challenging needs with regard to power consumption.

The above problem is solved by a cardiac pacemaker comprising the features of claim 1, an operation method of a cardiac pacemaker with the features of claim 8, a computer program product with the features of claim 14 and computer readable data carrier having the features of claim 15.

In particular, the above problem is solved by a cardiac pacemaker for a patient's heart, for example an ILP, comprising a processing unit, a detector and a pacing signal generator, wherein the processing unit, the detector and the pacing signal generator are electrically interconnected, wherein the detector is configured to detect electrical signals of the heart, for example an intracardiac electrogram (IEGM), and to transmit these signals to the processing unit, wherein the processing unit is configured to perceive an intrinsic ventricular signal and an intrinsic atrial signal from the signals received from the detector, to enable or disable the perception of the intrinsic atrial signal, to produce a ventricular pacing control signal comprising a ventricular pacing time information and to transmit the pacing control signal to the pacing signal generator for providing a pacing signal for the patient's heart, wherein the processing unit is configured to produce the ventricular pacing control signal using a VDD mode or using at least one supplementary mode, wherein the processing unit is configured to conditionally use
- the VDD mode in the current cardiac cycle, if perception of the intrinsic atrial signal is enabled, wherein pacing in the VDD mode is based on a current AV delay if an intrinsic atrial signal is perceived by the processing unit within the current cardiac cycle or based on a current VV delay determined from previous intrinsic ventricular and/or atrial signals if an intrinsic atrial signal is not perceived by the processing unit within the current cardiac cycle and
- alternatively, the at least one supplementary mode in the current cardiac cycle based on the current VV delay which is determined from a previous VV delay considering at least one additional parameter or from a different assignment rule.

The pacemaker may be a conventional cardiac pacemaker or an ILP having the general structure as indicated above and below. Further, generally the units and components may work in time rates or may use corresponding time intervals. Accordingly, below explanations with regard to time intervals shall be understood to analogously refer to rates and vice versa.

With regard to the invention, the processing unit is generally regarded as a functional unit of the pacemaker, that interprets and executes instructions comprising an instruction control unit and an arithmetic and logic unit. The processing unit may comprise or be a microprocessor, a controller, a digital signal processor (DSP), an application specific integrated circuit (ASIC), a field-programmable gate array (FPGA), discrete logic circuitry or any combination thereof. Alternatively or additionally, the processing unit may be realized using integrated dedicated hardware logic circuits, in particular in the case of an ILP due to the small size and extreme power limitation.

The processing unit processes signal data received from the detector, for example electrical signals of the patient's heart which are detected over time. In particular, the detector may be configured to detect the time-dependent electrical depolarization and repolarization field signals such as an electrocardiogram (ECG) or intracardiac (IEGM). These signals may comprise signals caused by the depolarization of the atria (in the following, the intrinsic atrial signal) and electrical signals caused by the depolarization of the ventricles (in the following, the intrinsic ventricular signal). In the case of an ILP the intrinsic atrial signal may be a far field electrical signal. The detector may preprocess these data, for example digitize the signals, filter them and/or amplify them.

The processing unit to which the electrical signals of the detector are transmitted perceives intrinsic ventricular signals and intrinsic atrial signals from the electrical signals, for example the intrinsic atrial signal from the P wave and the intrinsic ventricular signal from the QRS complex. Since the far-field electrically measured P wave is much smaller in amplitude compared to near-field ventricle signals, a higher amplification may be used in a time period of the signal in which the P wave is expected than in the QRS and T-wave time intervals.

The ILP or the conventional pacemaker is normally operated in the VDD pacing mode (i.e. a pacing mode in which the ventricle is stimulated according to atrial activity and AV conduction monitoring). In the VDD mode, the pacemaker synchronizes ventricular pacing with the intrinsic atrial signal based on a current AV delay. In an ILP that is implanted in the right ventricle, the atrial contraction information may be detected as a far field signal as indicated above.

The processing unit may further comprise a counter and a clock. In one embodiment the clock may operate at 128 Hz. The counter may be used to count clock signals of the clock. The counter may be started at each sensed atrial or ventricular depolarization and count the number of clock signals until the next atrial or ventricular depolarization occurs or ventricular pacing is provided by the pacing signal generator. In the VDD mode, the processing unit may determine from the intrinsic atrial signal and the intrinsic ventricular signal or the ventricular pacing signal the VV delay of the actual cardiac cycle which corresponds to the actual cardiac rate. For example, a "current (average) VV delay" may be determined as an average of previous VV delays of the previous cardiac cycles and of the VV delay of the actual cardiac cycle. In one embodiment, the VV delay of the actual cardiac cycle and the VV delays of previous cardiac cycles is/are only used to determine the current VV delay if at least one intrinsic signal (an intrinsic atrial signal or an intrinsic ventricular signal) was used to determine the VV delay of the respective cardiac cycle. The current VV delay may be used to provide the ventricular pacing control signal containing the ventricular pacing time, which may then be transmitted to the pacing signal generator.

Based on the pacing control signal, the pacing signal generator produces the electrical pacing signal(s) in order to transfer it to the electrodes which apply the signal(s) to the heart's tissue adjacent to the electrode. The pacing signals are pulses that begin at a desired time point and have a desired intensity and duration. Further, the pulse waveform may be varied. Information on the pacing signals, e.g. the ventricular pacing signals, that are necessary to produce the correct pacing signals are provided by the pacing control signal, e.g. by the ventricular pacing control signal, of the processing unit or by the pacing signal generator itself. In particular, the ventricular pacing control signal provides time information of the pacing signal, i.e. information on when the pacing signal shall immediately be provided to the patient's heart, e.g. the information that pacing shall be provided without further deferment. In one embodiment, the pacing signal is not determined (i.e. inhibited) or not transferred to the electrodes if an intrinsic ventricular signal is detected within a predefined time period (e.g. the AV delay) after the detected intrinsic atrial signal in the VDD mode.

The pacemaker may comprise a data memory which may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device. The data memory saves the above and below mentioned thresholds and conditions. They are required by the processing unit during processing the above and below explained steps.

The detector may further comprise an accelerometer, a vibration sensor, an acoustic sensor (including ultrasound) and/or any other mechanical, electric and/or magnetic sensor that is capable to detect the activity of the patient dependent on time (i.e. a motion sensor), e.g. whether the patient moves or moves not, for example lies, sleeps, sits, moves fast or slowly, including exercising. The detector collects the activity signals of the patient and transforms them into electrical signals. Further, the detector may digitize analog signals, filter them and/or smooth them in order to reduce signal noise. Some pre-processing steps may be provided by the detector, as well. The time dependent motion signal produced by the detector is preferably transmitted to the processing unit directly.

The pacemaker may comprise further modules such as a communication unit for communication with a remote computer and a power supply such as a battery. The communication unit may exchange messages with the external (at least partially extracorporeally) remote computer, for example in one single direction or bidirectionally. The communication may be provided wirelessly via the patient's body, preferably acoustic, conducted and/or magnetically coupled, and/or the air using electromagnetic waves, for example Bluetooth, WLAN, ZigBee, NFC, Wibree or WiMAX in the radio frequency region, or IrDA or free-space optical communication (FSO) in the infrared or optical frequency region or by wire (electrical and/or optical communication). The remote computer is a functional unit that can perform substantial computations, including numerous arithmetic operations and logic operations without human intervention, such as, for example, a personal mobile device (PMD), a desktop computer, a server computer, clusters/warehouse scale computer or embedded system. The pacemaker's units and components may be contained within a hermetically sealed housing.

In one embodiment the pacemaker comprises electrodes for application of an electrical pacing signal provided by the pacing signal generator. The electrodes are electrically connected to the pacing signal generator via a header of the pacemaker. In one embodiment (i.e. in the case in which the pacemaker is a conventional pacemaker) the electrode may comprise a lead which may be detachably connected to the respective connector at the header. With regard to an ILP one electrode may be located at a distal end of the ILP, close to a fixation member by which the ILP is fixed in the tissue of the patient's heart, for example against or within the tissue of a ventricle. A second electrode may be located at the proximal end of the ILP or a part of the ILP housing that may, for example, serve as counter electrode. Further, the electrodes may be adapted to detect the intrinsic ventricular signal or the intrinsic atrial signal in each case over time by picking up electrical potentials. The electrodes may thereby be part of the detector of the pacemaker.

The processing unit may be adapted to control pacing of the right ventricle normally in the VDD mode based on the perceived intrinsic atrial signal of the present cardiac cycle, if perception of the intrinsic atrial signal (i.e. atrial tracking) is enabled.

Normally, the pacing mode supported by the pacemaker is VDD. This mode assumes that the patient has some form of intrinsic AV block, either complete or intermittent. It also assumes that the sinus node is generally competent, and the VDD mode attempts to track the sinus rate and to provide AV synchronization. If an intrinsic atrial signal is revealed by the processing unit, a time interval "current AV delay" is started, for example by counting a respective counter counting clock signals down. If an intrinsic ventricular signal is perceived by the processing unit within the current AV delay, pacing is inhibited. If the AV delay expires without detecting an intrinsic ventricular signal a ventricular pacing control signal is produced and transmitted to the pacing signal generator thereby causing respective pacing of the patient's heart (except in the case of hysteresis). The current AV delay may be determined based on the current VV delay (see above) and may be set to the usual time difference between the intrinsic atrial and ventricular contraction or depolarization. From the current VV delay, the current AV delay may be determined by a known calculation or using a look-up-table contained in the data memory. The current AV delay changes with the current (average) VV delay. As indicated above, the VV delay represents the time between two consecutive ventricular contractions, wherein the contraction is either an intrinsic contraction or the contraction is produced by pacing.

However, in case no intrinsic atrial signal is determined within the current cardiac cycle in the VDD mode, then the ventricular pacing control signal is produced using the current VV delay. The down counting of the current VV delay starts with an immediately preceding perceived intrinsic ventricular signal or pacing signal. If an intrinsic atrial signal is seen, the counting of the AV delay prevails starting with the atrial signal. If no intrinsic atrial signal is detected, the current VV delay counting "steps in". If no intrinsic ventricular signal is seen, the pacing control signal is produced as soon as the current VV delay is expired causing corresponding pacing of the patient's heart. If an intrinsic ventricular signal is seen and the AV delay is not expired, pacing is inhibited. The same applies to the current VV delay (if no intrinsic atrial signal was perceived and therefore no AV delay is counted) - if the current VV delay is not expired and an intrinsic ventricular signal is seen, pacing is inhibited.

The qualification for using VV intervals to determine the current VV delay is that the measured VV intervals should only be used from VV intervals in which an intrinsic sense, either an atrial signal or a ventricular signal or both, is involved. When there is no intrinsic timing information in the cycle, the VV interval is not used to determine the current VV delay and thereby the pacing rate. If this cycle had an intrinsic atrial signal or ended in an intrinsic ventricular signal, then it was an intrinsic cycle. A cycle with an intrinsic atrial signal provides phase information, but not necessarily rate information. Two intrinsic cycles in a row provide also rate information and thereby qualify to use the VV interval for determination of the current VV delay.

If certain pre-defined conditions apply, the processing unit exits the VDD mode and uses at least one supplementary mode for pacing. For example, the VDD mode may be exited or not used if synchronization with the intrinsic atrial and/or ventricular signals has been lost since a pre-defined time or number of cardiac cycles (i.e. no atrial and/or ventricular signals were perceived by the processing unit), if the actual cardiac rate (determined VV delay of the actual cardiac cycle or of the immediately preceding cardiac cycle) is greater than a pre-defined threshold, or if a transition back to the VDD mode is attempted. For the at least one supplementary mode a current VV delay is determined from a previous VV delay considering at least one additional parameter or from a different assignment rule. The different assignment rule may be based on signals of a sensor other than the intrinsic atrial or ventricular signals, e.g. motion signals, or on setting the current VV delay to a pre-defined value or on ramping the current VV delay to a pre-defined target value.

In one embodiment, a first supplementary mode is a VVI mode and a second supplementary mode is a sensor mode, wherein in the VVI mode the processing unit is configured to determine the current VV delay from a basic rate, wherein in the sensor mode the processing unit is configured to determine the current VV delay based on signals of a sensor of the detector detecting signals different from the intrinsic ventricular signals and the intrinsic atrial signals, for example motion signals, if perception of corresponding sensor signals is enabled. Accordingly, the sensor mode is similar to a VVI(R) mode in which the current VV delay is determined, for example, by adaption of the current VV delay based on the dynamics of the patient's motion. Whether the first supplementary mode or the second supplementary mode is used depends on whether second supplementary mode is enabled by the respective programming or pre-defined setting. The basic rate is the minimal rate a patient shall have during pacing. The current VV delay from the basic rate is determined as the interval corresponding to the basic rate. The current VV delay in the second supplementary mode is determined dependent from the intensity of patient's motion perceived from the sensor's motion signal. If no motion is detected, the current VV delay is determined using the resting rate. If the patient's motion has a certain intensity the corresponding current VV delay may be determined using a specific calculation or stored values of a look-up-table. As indicated below, the current VV delay may be determined by ramping the VV delay up or down, wherein the VV delay value corresponding to the actual motion intensity of the patient is the target value, if the difference to the VV delay of the actual cardiac cycle is too big. The same applies analogously to other sensor signal different from motion signals. In one embodiment in the sensor mode perception of intrinsic atrial signals is disabled, i.e. atrial tracking is disabled, because it has a certain energy demand. Further, for the sensor mode the sensor is configured to detect the corresponding signals.

In this embodiment, in the pacemaker, for example in an ILP, the VDD mode and one of the first and second supplementary mode is generally mutually exclusive because the power budget to do both would be prohibitive for a device with such a small battery. Both modes, the VDD mode and the supplementary mode, are thus treated as complimentary timing mechanisms. The second supplementary mode works more reliably at higher rates where VDD mode is problematic because of merging in time of the atrial signals with larger amplitude of ventricular signals. The VDD mode provides AV synchrony and is thus more hemodynamically efficient.

In one embodiment, if in the VDD mode or the second supplementary mode an intrinsic ventricular signal is not perceived within the time interval of the current VV delay, the current VV delay may be prolonged by a first hysteresis delay, wherein this prolongation is provided over a pre-defined number of consecutive cardiac cycles without any perceived intrinsic ventricular signal. The hysteresis delay may be, for example, between 50 ms and 100 ms. The pre-defined number on consecutive cycles without any perceived intrinsic ventricular signal may be between 3 and 10. Both values may be hardcoded or programmable by the HCP using the programmer. By the prolongation of the VV delay automatically pacing rate is reduced because a pacing control signal is produced by the processing unit at a time point at which the VV delay prolonged (extended) by the hysteresis delay is expired and no intrinsic ventricular signal was sensed in the meantime (i.e. within this cardiac cycle). It is assumed that a sudden drop of intrinsic heart rate or loss of AV conduction has occurred.

In one embodiment the processing unit comprises a hysteresis component which provides the first hysteresis delay for the VDD mode and the second supplementary mode (the sensor mode). Alternatively or additionally a second hysteresis delay for the VDD mode for prolongation of the AV delay may be provided by the hysteresis component. The second hysteresis delay may be equal to or different from the first hysteresis delay. It may be, for example, between 50 ms and 100 ms. The second hysteresis delay prolongs the AV delay such that a ventricular pace (and a pacing control signal) is only be provided if the AV delay is prolonged (extended) by the second hysteresis delay is expired and no intrinsic ventricular signal has been sensed within this cardiac cycle. This encourages ventricular sensing in the VDD mode. The above mentioned hysteresis delay values may be shorter than typical AV hysteresis values used in DDD pacemakers to avoid unnecessary ventricular pacing. In an atrial tracking states in the VDD mode, the interval source is the average current ventricular interval (VV delay). The atrial signal in a ventricular ILP that does AV synchronization is a far-field signal that may be qualified by where it occurs within the cardiac cycle to avoid being overwhelmed by ventricular artifacts. Such sense detections will inherently be unavailable on some cardiac cycles. If in an atrial tracking state in VDD, missed atrial senses can be "filled in" or "compensated for" by using the recently averaged measured VV interval (current VV delay) as the pacing rate (see explanation above). If intermittent intrinsic AV conductions are occurring, a hysteresis extension by the second hysteresis delay may help avoiding competitive pacing on cardiac cycles with AV conductions. The hysteresis component may comprise a hysteresis logic component which is used to keep track of whether the pacemaker should use hysteresis extensions like the first hysteresis delay and the second hysteresis delay on any given cycle. The hysteresis logic component therefore comprises a counter to count the consecutive cardiac cycles without any intrinsic ventricular signal. Additionally, the hysteresis component may comprise a hysteresis timer component for providing the correct time of the first hysteresis delay and the second hysteresis delay at the end of the AV delay or the current VV delay, respectively, if no intrinsic ventricular signal occurred in the respective cardiac cycle so far.

In one embodiment, the processing unit is configured such that it uses a first subsequent mode, namely the rate fading mode, starting from the next cardiac cycle following the pre-defined number of consecutive cardiac cycles (i.e. the next cardiac cycle directly follows the pre-defined number of consecutive cycles) in which the prolongation of the current VV delay is provided by the first hysteresis delay and no intrinsic ventricular signal is perceived in the VDD mode or the second supplementary mode, wherein in the rate fading mode the VV delay is ramped up from the current VV delay until a VV delay corresponding to a basic rate or a current sensor rate is reached (i.e. the rate corresponding to the VV delay is ramped down). This means that the basic rate and the current sensor rate, respectively, forms the target rate for the rate fading process. This embodiment is based on the consideration that after the pre-defined number of consecutive cycles without any detected intrinsic ventricular signal (the number may be, for example between 3 and 10, counted by the counter mentioned above) the current VV delay is not applicable to the present situation of the patient, it must be considered out-of-date. Accordingly, the processing unit ramps the VV delay up (or the pacing rate down) as it seems that still there is a drop of the intrinsic heart rate. The rate (corresponding to the VV delay) may be ramped down by approximately 0.5 bpm (decrement rate change value) in each step (e.g. each cardiac cycle), for example by using the rate limiter component as described below. This may be accomplished by using three different slopes (based on the resolution of 7.8125 ms or 128 Hz) in different rate regions. If the current VV rate is 40 or less, the decrement value is the current VV delay divided by 8 and minus 16, if the rate is greater than 40 but less than 80, the decrement value is the current VV delay divided by 16 and minus 4. If the rate is equal to or greater than 80, the decrement value is the current VV delay divided by 32 minus 1.

Alternatively or additionally, the rate interval may be incremented in 7.8125 ms units by adding 1/64 of the current interval to the current interval (plus one extra unit if the rate is below 120 bpm).

As indicated above, the basic rate is the lowest pacing rate that should be used for the patient. The resting rate is the rate which the patient should have if the patient rests. The resting rate is usually higher than the basic rate. In one embodiment the basic rate is used as the target rate for the VDD mode and the current sensor rate is the target rate for the second supplementary mode (sensor mode). The lowest sensor rate is the resting rate. If the current motion signal received by the processing unit indicates that the patient is not at rest but moving the sensor rate is correspondingly greater. The basic rate and/or the resting rate may be pre-defined or programmable by the HCP for the specific patient, for example by using the programmer.

Rate fading is a subsequent mode that recognizes when a sudden intrinsic rate drop has occurred. In the VDD mode which uses the VV delay, the sudden rate drop can be the result of either a failure of the sinus node or the AV conduction path. In cases in which atrial tracking is supported, AV conduction failures are being handled by the atrial tracking algorithm as indicated above, but sinus rate failures can still result in sudden intrinsic rate drops. In one embodiment, sinus rate drops while in an atrial tracking state may be handled by recognizing the absence of intrinsic senses and switching to a non-atrial tracking mode (VDD mode with VV delay tracking, first or second supplementary mode). This means that during the rate fading mode perception of intrinsic atrial signals is disabled. For power and space efficiency, this feature is realized as described above by using the rate management known from the sensor mode with some small additions (see above).

In one embodiment the processing unit will switch into a second subsequent mode, namely a FindSync mode, in which the processing unit seeks to perceive intrinsic atrial signals and atrial tracking opportunities if in the rate fading mode or in the sensor mode the current VV delay corresponds to the basic rate or the resting rate for a pre-defined time interval. The FindSync (short for "find synchronization") state defines a behavior that provides basic support while attempting to encourage detection of intrinsic activity. It is a hybrid state between atrial-tracking (VDD) and non-atrial tracking (VVI) states. In the case where there are no intrinsic senses, it provides an asynchronous (i.e. atrial and ventricular intrinsic signals are not being synchronized) mode in which the pacing rate is the resting rate, independent of what the undetected intrinsic heart rate might be. When intrinsic atrial signals are detected, they start an AV delay (determined from the resting rate) which helps phase shift the pacemaker activity to align it with the cardiac activity. Detection of a single intrinsic atrial signal does not provide sufficient information to allow rate matching between the pacemaker and the heart. If two consecutive cardiac cycles both include either intrinsic atrial or ventricular signals, the VV interval between these cycles can be measured and this measurement may be used as the VV delay (corresponds to the pacing rate) so that both the phase and rate of the heart and pacemaker will be aligned, thus ensuring that ventricular pacing can be delivered in synchronization with the intrinsic heart's signals. Conditions that indicate that pacing is locked to the intrinsic rate will take the pacemaker back to the VDD mode.

In one embodiment the processing unit comprises a rate limiter component which determines the current rate for ramping the current VV delay in the rate fading mode or for adaption of the current VV delay to the detected sensor signals in the sensor mode, wherein the rate limiter component provides a fixed attack rate change value and the fixed decrement rate change value defined above, which define the step sizes in attack and decay direction, respectively. An example for the decrement rate change value is provided above the attack rate change value may be, for example, 2 bpm. Both rate change values may be pre-defined in the pacemaker or user-selectable by the HCP using the programmer. The attack rate change value may be approximated by dividing the current VV delay by 64.

It was indicated above that the rate change limitation may be provided for ramping the current VV delay (which corresponds to the pacing rate) in the rate fading mode. Additionally, the rate change mechanism (or analogously an interval change mechanism) may be used for adaption of the VV delay to the current motion signal received by the processing unit. Analogous to the VVI(R) mode the time interval of the current VV delay will be changed 1) to longer intervals if the current motion signal indicates a reduced movement of the patient, 2) to the time interval corresponding to the resting rate if the motion signal indicates that the patient is at rest or 3) to shorter intervals if the current motion signal indicates an increased movement of the patient.

The rate limiter component is responsible for providing rate transitions where rate changes are limited to a pre-defined attack rate change value or decrement rate change value. The destination rate for the transition is called the target rate. Each step towards the target is at a next rate and steps occur at each cardiac cycle. The rate limiter component may work in intervals rather than in rates. The attack and decrement change intervals may be rate dependent. The max delta (either up or down) for the next cardiac cycle interval is calculated by the rate limiter component based on the current VV delay. In one embodiment, the current VV delay applicable for rate transition may be limited to an upper tracking interval limit. The current VV delay is compared by the rate limiter component to the target interval to see if it needs to speed up or slow down the rate in order to move towards the target interval at the maximum rate of change allowed. The next interval output is determined from the current rate and the largest step allowed in one cardiac cycle. An embellishment or a consequence of this attack/decay limiting component is to prevent oscillations when following/approaching target rates. The step sizes allowed are typically greater than the resolution of the target interval. To prevent such oscillations, which could needlessly use extra power from the battery, the logic of the rate limiter component may keep track of when the target value crosses over from attacking to decaying or the other way. Once this transition occurs, the next step can be set to exactly the target interval. For correct usage the rate limiter component may be first be initialized so that the current VV delay is set to the current pacing interval, i.e. the last interval used in the previous mode or in the previous state of the mode (if, for example, the motion activity of the patient has changed). This provides an automatic gradual transition from the old sourced interval to the new sourced interval. The rate limiter component may be used in the sensor mode or in the rate fading mode.

In order to adapt the rate limiter component to the different modes of the processing unit, an interval source (rate source) may be selected for the target interval (target rate). This may be provided by a rate limiter target select component of the processing unit. Possible sources for this selection may include a sensor derived interval (such as from the motion sensor in the sensor mode corresponding to the actual motion intensity of the patient) or an interval corresponding to a lower rate such as the resting rate or the basic rate in the rate fading mode. The selection of the target interval is provided based on the present mode of the pacemaker.

In one embodiment, the processing unit comprises a rate multiplexer component which selects the appropriate pacing rate, which corresponds to the current VV delay, according to the present mode of the processing unit. If certain conditions apply in the sensor mode, the VVI mode, the rate fading mode or the FindSync mode a lower rate may be selected such as the resting rate or the basic rate. In the VDD mode the (averaged) current VV delay is selected. Further, in the sensor mode or in the rate fading mode an VV delay (interval) corresponding to a rate determined by the rate limiter component may be selected.

In one embodiment, the processing unit is configured such that the sensor mode and/or the VVI mode may be chosen by the HCP using the programmer as the normal mode. Accordingly, atrial tracking is not allowed. The processing unit is adapted to produce a ventricular pacing control signal based on the above described sensor mode or VVI mode, respectively. The processing unit does not use the VDD mode in this clinician-chosen condition and accordingly, the FindSync mode is not enabled for atrial tracking, too.

The above problem is further solved by an operation method of a cardiac pacemaker for a patient's heart, for example an ILP, comprising a processing unit, a detector and a pacing signal generator, wherein the processing unit, the detector and the pacing signal generator are electrically interconnected, wherein electrical signals of the heart, for example an intracardiac electrogram (IEGM), are detected by the detector and transmitted to the processing unit for perception of an intrinsic ventricular signal and an intrinsic atrial signal from the signals received from the detector, wherein the perception of the intrinsic atrial signal can be enabled or disabled by the processing unit, wherein a ventricular pacing control signal comprising a ventricular pacing time information is produced by the processing unit and transmitted to the pacing signal generator for pacing signal generation for the patient's heart, wherein the ventricular pacing control signal is produced conditionally using a VDD mode or at least one supplementary mode, wherein
- the VDD mode is used in the current cardiac cycle, if perception of the intrinsic atrial signal is enabled, wherein pacing in the VDD mode is based on a current AV delay if an intrinsic atrial signal is perceived by the processing unit within the current cardiac cycle or based on a current VV delay determined from previous intrinsic ventricular and/or atrial signals if an intrinsic atrial signal is not perceived by the processing unit within the current cardiac cycle and
- alternatively, the at least one supplementary mode is used in the current cardiac cycle based on a current VV delay determined from a previous VV delay considering at least one additional parameter or from a different assignment rule.

In one embodiment of the method a first supplementary mode is a VVI mode and a second supplementary mode is a sensor mode, wherein in the VVI mode the current VV delay is determined from the basic rate, wherein in the sensor mode the current VV delay is based on signals of a sensor of the detector detecting signals different from the intrinsic ventricular signals and the intrinsic atrial signals, for example motion signals, if perception of corresponding sensor signals is enabled..

There is one embodiment of the method wherein, if in the VDD mode or the second supplementary mode an intrinsic ventricular signal is not perceived within the time interval of the current VV delay, the current VV delay is prolonged by a first hysteresis delay, wherein this prolongation is provided over a pre-defined number of consecutive cardiac cycles without any perceived intrinsic ventricular signal, wherein additionally or alternatively a second hysteresis delay for prolongation of the AV delay is used in the VDD mode, wherein the first hysteresis delay and/or the second hysteresis delay may be provided, for example, by a hysteresis component of the processing unit.

In one embodiment of the method a first subsequent mode is used, namely a rate fading mode, starting from the next cardiac cycle following the pre-defined number of consecutive cardiac cycles in which the prolongation of the VV delay is provided by the first hysteresis delay and no intrinsic ventricular signal is perceived in the VDD mode or in the second supplementary mode, wherein in the rate fading mode the VV delay is ramped up from a current VV delay to a VV delay corresponding to a basic rate or a current sensor rate.

In one embodiment, the method will switch into a second subsequent mode, namely a FindSync mode, in which the processing unit seeks to perceive intrinsic atrial signals and atrial tracking opportunities if in the rate fading mode or in the sensor mode the current VV delay corresponds to the basic rate or a resting rate for a pre-defined time interval.

In one embodiment, the current rate for ramping the VV delay is determined in the rate fading mode or for adaption of the current VV delay to the detected sensor signals in the sensor mode by a rate limiter component, wherein the rate limiter component provides an attack rate change value and a decrement rate change value.

The above embodiments of the operation method have the same advantages as the above pacemaker. Embodiments of the pacemaker indicated above may be realized in the operation method analogously. It is referred to the above explanation of the pacemaker in this regard.

The above method is, for example, realized as a computer program which comprises instructions which, when executed, cause the processing unit (processor) to perform the steps of the above method (to be executed by the cardiac pacemaker, in particular at its processing unit) which is a combination of above and below specified computer instructions and data definitions that enable computer hardware to perform computational or control functions or which is a syntactic unit that conforms to the rules of a particular programming language and that is composed of declarations and statements or instructions needed for a above and below specified function, task, or problem solution.

Furthermore, a computer program product is disclosed comprising instructions which, when executed by the processing unit, cause the processing unit to perform the steps of the above defined method. Accordingly, a computer readable data carrier storing such computer program product is disclosed.

The above pacemaker, method, computer program and computer program product provide a common rate management design that implements different programmed pacing modes and manages dynamic supplementary and subsequent modes. Further, the inventive solution provides dynamic selection of rate (interval) source based on programmed mode conditions, rate source driven control of rate change limitation or instantaneous rate changes, integration of rate source selection and rate smoothing functionality, sharing of a common mechanism for rate limiting and rate ramping for different modes and use of hysteresis extension (hysteresis delay) for triggering of rate fading behaviour.

The present invention will now be described in further detail with reference to the accompanying schematic drawing, wherein
- Fig. 1: shows a first embodiment of the pacemaker within a cross section of a patient's heart,
- Fig. 2: depicts a functional block diagram of the pacemaker shown in Fig. 1,
- Fig. 3: shows an enlarged side view of the pacemaker of Fig. 1,
- Fig. 4: depicts a block diagram showing components of the processing unit visualizing the pacemaker timing and rate management in different modes of the pacemaker of Fig. 1,
- Fig. 5: shows the rate behavior over time in the VDD mode (without atrial tracking) around a sudden drop in the cardiac rate provided by the processing unit of the pacemaker of Fig. 1, and
- Fig. 6: shows the rate behavior over time in the sensor mode around a sudden drop in the cardiac rate provided by the processing unit of the pacemaker of Fig. 1.

In the following the invention is described with regard to an ILP. It may analogously be realized in a conventional pacemaker, as well.

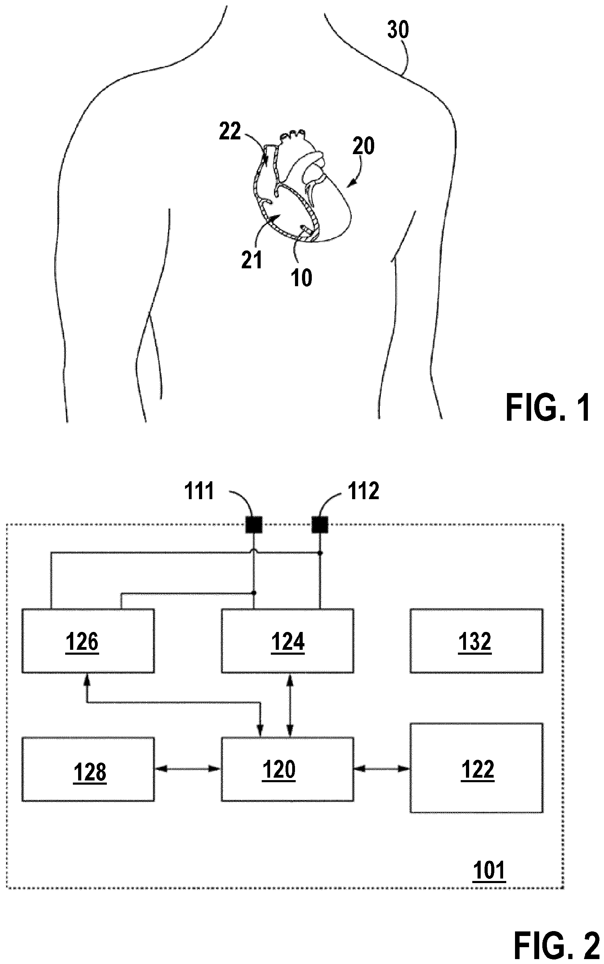

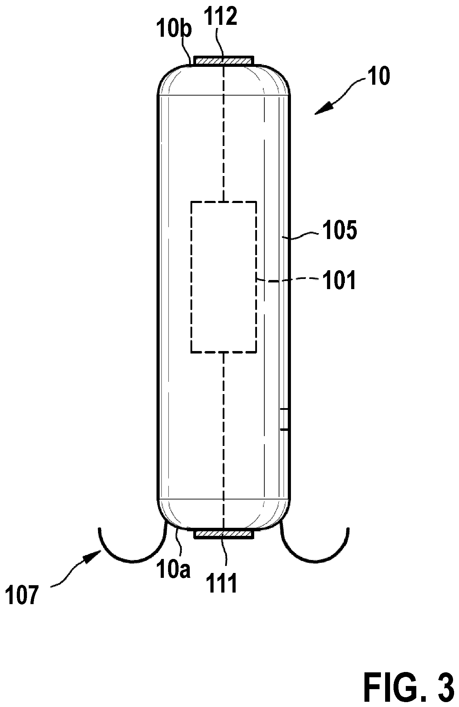

Fig. 1 shows an example leadless ventricular pacemaker (ILP) 10 implanted within the heart 20 of a patient 30. The exemplary ILP 10 is depicted in Fig. 3 in an enlarged view. ILP 10 has a distal end 10a and a proximal end 10b and may be configured to be implanted within the right ventricle 21 of the heart 20 and pace this ventricle, sense intrinsic ventricular depolarizations and intrinsic atrial depolarizations (e.g. the right atrium 22), and inhibit ventricular pacing in response to detected intrinsic ventricular signal in the VDD mode. A programmer (not shown) may be used to program ILP 10 and retrieve data from ILP 10. The ILP 10 is one example of a cardiac pacemaker 10. Other embodiments of the cardiac pacemaker 10 are possible.

Fig. 2 shows a functional block diagram of circuitry 101 of the ILP 10 configured for implantation within ventricle 21 (Fig. 1). The circuitry 101 of ILP 10 comprises a processing unit 120 with a clock providing a clock signal preferably between 100 and 200 Hz, particularly preferred of 128 Hz, counters for the clock signals, registers and a data memory 122 that may include registers, a pacing signal generator 124, a detector 126, a communication unit 128, and a power source 132. The power source 132 may be electrically connected to one or more of the other components 120, 122, 124, 126, 128 (not shown in Fig. 2) and may include a battery, e.g., a rechargeable or non-rechargeable battery. The power source provides electrical energy to all units and components of the ILP 10, in particular to all units mentioned above and is therefore electrically connected to these units and components. The mentioned units included in ILP 10 represent their respective functionality. Similar or identical units and functionality may also be included in the ILP 10. Units of the pacemaker of present disclosure may include any discrete and/or integrated electronic circuit components that implement analog and/or digital circuits capable of producing the functions attributed to the units herein. For example, the units may include analog circuits, e.g., amplification circuits, filtering circuits, and/or other signal conditioning circuits. The units may also include digital circuits, e.g., combinational or sequential logic circuits, memory devices, etc. The units may be further realized using integrated dedicated hardware logic circuits. The data memory 122 may include any volatile, non-volatile, magnetic, or electrical media mentioned above. Furthermore, the processing unit 120 may include instructions that, when executed by one or more processing circuits, cause the units to perform various functions attributed to these units herein. The functions attributed to the units or component herein may be embodied as one or more processors, hardware, firmware, software, or any combination thereof. Depiction of different features as units or components is intended to highlight different functional aspects, and does not necessarily imply that such units must be realized by separate hardware or software components. Rather, functionality associated with one or more units or components may be performed by separate hardware or software components, or integrated within common or separate hardware or software components. Data memory 122 may store computer-readable instructions that, when executed by processing unit 120, cause processing unit 120 to perform the various functions attributed to processing unit 120 herein. Further, data memory 122 may store parameters for these functions, e.g. pacing signal parameters, conditions and thresholds described above and below. The pacing instructions and pacing signal parameters, conditions and thresholds may be updated by the programmer using the communication unit 128. The communication unit 128 may comprise an antenna or a transceiver.

The processing unit 120 may communicate with pacing signal generator 124 and detector 126 thereby transmitting signals. Pacing signal generator 124 and detector 126 are electrically coupled to electrodes 111, 112 of the ILP 10. Detector 126 is configured to monitor signals from electrodes 111, 112 in order to detect the electrical activity of heart 20. Further, the detector 126 may include a motion sensor, for example an accelerometer or any other motion sensor described above. The motion sensor collects a time-dependent motion signal as described above and transmits this signal to the processing unit 120. Pacing signal generator 124 is configured to deliver electrical stimulation signals to ventricle 21 via electrodes 111, 112. Processing unit 120 may control pacing signal generator 124 to generate and deliver electrical stimulation to ventricle 21 via electrodes 111, 112. Electrical stimulation may include pacing pulses.

The electrode 112 is located where a mechanical hitch resides which means, in a preferred or practical built embodiment, the electrode 112 is not easily be located at the proximal most end (as shown in Fig. 3). The better approach would be to present a ring electrode near the proximal end, but not at the absolute extreme terminus of the device.

Processing unit 120 may control pacing signal generator 124 to deliver electrical stimulation therapy according to one or more therapy programs including pacing parameters, which may be stored in data memory 122 or may be hardcoded in the processing unit 120.

Detector 126 may further include circuits that acquire time-dependent electrical signals (e.g. electric depolarization and repolarization signals) from the heart including intrinsic cardiac electrical activity. Detector 126 may filter, amplify, and digitize or otherwise preprocess the acquired electrical signals of the heart chambers contractions. Processing unit 120 may receive the intrinsic electrical signals generated by detector 126 and perceive the intrinsic atrial signals and intrinsic ventricular signals of the patient's heart.

Processing unit 120 may asses the intrinsic atrial signal and the intrinsic ventricular signal received from the detector 126 and is configured to determine the intrinsic interval of two consecutive ventricular signals (at least one is intrinsic) or an intrinsic AV interval (interval between an intrinsic atrial signal and the subsequent ventricular signal).

ILP 10 may include a housing 105, fixation tines 107, and the electrodes 111, 112. The housing 105 may have a pill-shaped cylindrical form factor in some examples. Fixation tines 107 are configured to connect (e.g., anchor) ILP 10 to heart 20. Fixation tines 107 may be fabricated from a shape memory material, such as Nitinol. In some examples, fixation tines 107 may connect ILP 10 to heart 20 within one of the chambers of heart 20. For example, as illustrated and described herein with respect to Fig. 1, fixation tines may be configured to anchor ILP 10 to heart 20 within right ventricle 21. Although ILP 10 includes a plurality of fixation tines 107 that are configured to anchor ILP 10 to cardiac tissue in the right ventricle, it is contemplated that a pacemaker according to the present disclosure may be fixed to cardiac tissue in other chambers of a patient's heart 20 using other types of fixation mechanisms.

ILP 10 may include two electrodes 111, 112, although more than two electrodes may be included on a pacemaker in other examples. Electrodes 111, 112 may be spaced apart a sufficient distance to be able to detect various electrical signals generated by the heart 20, such as P-waves generated by atria and QRS complex generated by ventricles. For example, the first electrode 111 is located at the distal end 10a of the ILP 10 and the second electrode 112 is located at the proximal end 10b of the ILP 10. The housing 105 houses electronic components (circuitry 101) of ILP 10. Electronic components may include any discrete and/or integrated electronic circuit components that implement analog and/or digital circuits capable of producing the functions attributed to ILP 10 described above and below.

The communication unit 128 of circuitry 101 may enable ILP 10 to communicate with other electronic devices, such as a programmer or other external patient monitor. In some examples, the housing 105 may house an antenna or transceiver for wireless communication. Housing may also include the power source 132.

The processing unit 120 may be adapted to control pacing of the right ventricle 21 in the VDD mode based on the intrinsic atrial signal containing atrial contractions and the intrinsic ventricular signal indicating ventricular contractions. Alternatively, an supplementary mode may be used by the processing unit 120 if the VDD mode turns out to be not appropriate for the individual patient in the present situation, for example with regard to the detected electrical signals or the motion state of the patient.

The processing unit 120 provides the synchronization and timing for ventricular pacing and inhibition based on clinical programming and the sensed intrinsic timing from the heart (e.g. sensed electrical signals from the heart) provided by detector 126. Additionally, the detector 126 may provide detected time dependent motion signals and transmit them to the processing unit 120.

In one embodiment, a first supplementary pacing is a (non-atrial tracking mode) similar to a VVI mode and in a second supplementary mode is a sensor mode similar to a VVI(R) mode. Other supplementary pacing modes (e.g. VOO) may be supported as test mode or with regard to different situation. The sensor mode may be used only if the motion-based rate response is enabled (for example by the HCP using the programmer).

In the VDD mode, the atrial tracking mode, intrinsic cardiac timing can be tracked if either atrial or ventricular senses, or both, are detected. If atrial senses are detected, they resynchronize the cycle by starting an AV delay, wherein the AV delay is determined using the current VV delay and a look-up table contained in the data memory 122. The next cycle is started when either a ventricular sense is detected or when a ventricular pace is delivered at the end of the AV delay. Allowing AV conduction to prevail when it is occurring often allows for improved hemodynamics even at the expense of having longer than normal AV intervals. A second hysteresis delay described above may be used to prolong the AV delay in order to support scanning for and to persist intrinsic AV conductions that might otherwise be hidden by pacing using the standard timing according to the AV delay.

The VDD pacing mode in the preferred normal mode may be R-Synchronization in the ILP 10. This means that every cycle is synchronized by every used ventricular event (i.e. by intrinsic ventricular contraction or ventricular pacing). The time interval between ventricular events is measured (VV intervals). In the VDD mode atrial tracking is provided. This means that every sensed atrial contraction can shift the timing. In other words, the VDD mode is both R-synchronized and P-synchronized. The timing of the next potential ventricular pacing signal is scheduled based on the most recent ventricular event and a pacing interval (e.g. the VV delay). Sensed atrial contractions "re-schedule" the next pacing signal by starting an AV interval.

A first supplementary mode can be used by the ILP for pacing which works similar to VVI pacing. Alternatively, if enabled by the HCP, a sensor mode may be used to which the processing unit 120 automatically switches if AV synchronization is not achieved over a longer time interval (for example 5 to 10 cycles without any detected intrinsic atrial or ventricular signal). The sensor mode operates similar to the VVI(R) pacing and uses the signals provided by the motion sensor of the detector. If the motion sensor indicates the patient needs only resting rate cardiac support, the algorithm of the processing unit 120 may be configured such that it, e.g. after some time, automatically returns to the VDD mode and tries to establish AV synchronization in the subsequent FindSync mode.

The VDD mode uses continuously measured qualified VV intervals to determine the pacing interval (current VV delay). Because the pacing rate matches the intrinsic rate, there is a race condition in which a ventricular signal of the pace and an intrinsic ventricular signal of one cardiac cycle are expected at roughly the same time. When there is no intrinsic AV conduction (3rd degree block), there is no competition. When intrinsic AV conduction is reliable, the heart should win. This can be handled by providing a AV delay hysteresis (which may be selected by the HCP). If there have been recent intrinsic ventricular signals and the pacemaker is detecting intrinsic atrial signals, the phase alignment of the ventricular paces with respect to the atrial intrinsic signals is shifted by making the AV delay longer than the programmed AV delay. This is realized by a second hysteresis delay which is added to the programmed AV delay (i.e. AV hysteresis is active) so that intrinsic ventricular signals are encouraged.

If intrinsic ventricular signals are not observed by the processing unit over a longer time consecutively, the processing unit will ramp the pacing rate (VV delay) down to the resting rate which is then the target rate. The resting rate is expected to be the patient's normal not active rate as it is indicated above. In a rate fading mode the pacing rate transitions from the initial rate to the target rate by stepping down using steps of a decrement rate change value.

The subsequent FindSync mode defines a behavior that provides basic support while attempting to encourage detection of intrinsic activity as indicated above. As indicated above the FindSync mode provides an asynchronous mode in which the pacing rate (VV delay) is the resting rate. In the FindSync mode perception of intrinsic atrial and ventricular signals is enabled. If two consecutive cardiac cycles both include either atrial or ventricular intrinsic signals, the VV interval between these cycles can be measured and this measurement can be used to set the VV delay and the start time of the cardiac cycle so that both the phase and rate of the heart and pacemaker will be aligned.

In the second supplementary mode the motion sensor is active and the timing system ignores intrinsic atrial signals. The processing unit 120 operates effectively in a VVI(R) mode. When the motion sensor rate drops down to the resting rate, the system switches back to the FindSync mode and begins monitoring for intrinsic activity again.

In this embodiment all timing and measurements are done with a resolution of 7.8125ms (1 tick at 128 Hz). This can time down to a rate of 30 bpm using a 1-byte counter. It is also the resolution for AV delays. The ILP 10 may have a maximum rate of 170 bpm. Calculated timer values are limited by the hardware to ensure that pacing does not occur faster than 170 bpm.

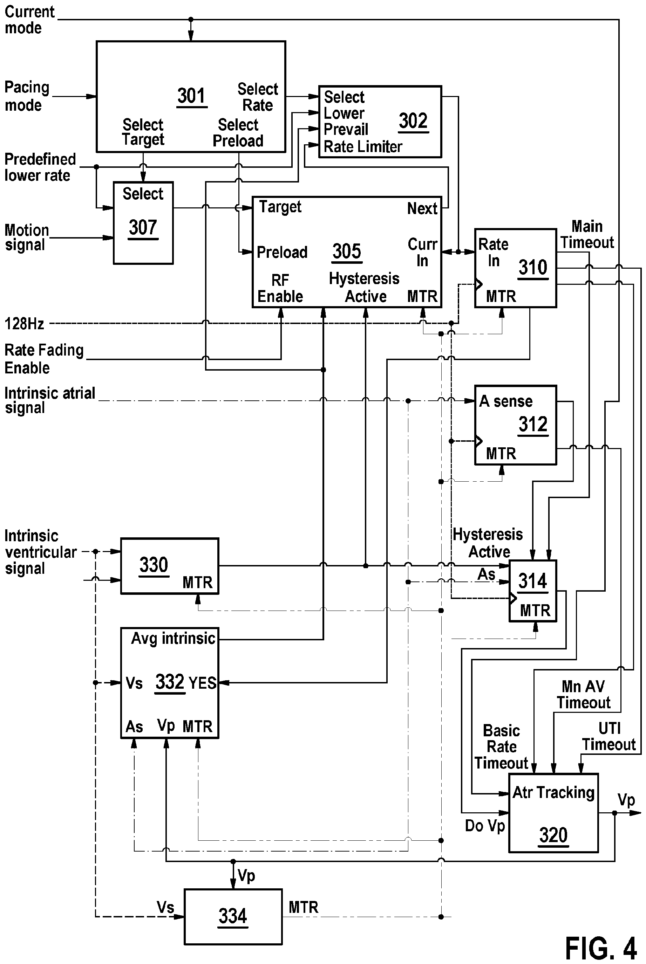

The processing unit 120 comprises timers which are up counters with comparators allowing the same counter to time several events that all are referenced to the same starting point. In general, the timers are all synchronized (i.e. started over) by a common reset called the Main Timer Reset, or MTR, however only the Main Timer continues counting when they are reset. The block diagram of Fig. 4 shows the main interactions between components of the processing unit 120. The inputs consist of the detector 126 outputs and pre-defined or programmed parameter values saved and read from the data memory 122 that provide the main configurability of the timer system. The only output relative to pacing is the ventricular pace output, i.e. the ventricular pacing control signal. Additionally, the timer system may be involved in measuring time intervals for statistics.

The subsystem of the processing unit referring to the timing may be controlled by registers and the mode of operation may be read in a series of registers. The HCP configures the ILP 10 by configuring screen parameters and transmitting the corresponding program parameter set. The registers all have reset modes that become active when a system or initial power up reset is triggered.

Controlling of the used modes of the processing unit is provided by enable bits. The mode of the processing unit is chosen according to the value of these bits. If an As enable bit is low, intrinsic atrial signals are masked and the clock to the AV delay timer will never be started. Then, the first supplementary mode (VVI(R) mode) is used, for example. If an intrinsic ventricular signal enable bit is low, ventricular intrinsic signals are masked and do not cause any main timer reset. If the intrinsic atrial signals enable bit is high, the intrinsic ventricular signal enable bit is high, the pacing bit is high, the processing unit operates in the normal VDD mode.

The method shown in diagram of Fig. 4 comprises controlling the pacing interval source, the tracking interval behavior, the motion circuit (enabled/disabled), the atrial tracking behavior (atrial sensing enabled/disabled), and the rate fading behavior under the various conditions that may arise while the pacemaker is programmed to the VDD mode. The ideal behavior in the VDD mode is to have the ventricular event (either an intrinsic ventricular contraction or a ventricular pacing signal if the intrinsic AV conduction is inadequate) track the intrinsic atrial contractions. If an intrinsic ventricular contraction occurs before the AV delay timeout, the pacemaker stops the AV delay and waits for the next atrial depolarization provided by the atrial intrinsic signal. If the AV delay timeout occurs, a ventricular pacing control signal is produced and based on this a ventricular pacing signal is delivered.

The diagram comprises a rate multiplexer component 302 which provides the rate source (actually the interval source) as an output which is selected by the control logic component 301 (see input of the rate multiplexer at the upper left corner and the inputs "Lower", "Prevail" and "Rate Limiter"). In other words: The rate multiplexer component 302 selects one of the rates at the input for the pacing within the actual cardiac cycle. The output of the rate multiplexer component 302 is used as an input to one of the Main Timer component's 310 comparators. The lower rate input register ("Lower" input) is programmed with either the basic rate (for the first supplementary mode) or the resting rate (for VDD mode or sensor mode) at the time that the pacing mode is programmed. The rate at the "Prevail" input is the (average) current VV interval provided by the interval monitor component 332 for the VDD mode. The "Rate Limiter" rate is provided by the rate limiter component 305 for the sensor mode (second supplementary mode) or the rate fading mode.

The control logic component provides a select signal for the rate multiplexer component 302 based on the programmed pacing mode (VDD or VVI(R), for example) and the information on the current mode. The current mode is the normal pacing mode (e.g. VDD) or one of its supplementary modes or one of its subsequent modes defined above.

As explained above the rate limiter component 305 is responsible for providing rate transitions where the rate changes are limited to a pre-determined attack rate change value or a pre-determined decrement rate change value (sensor mode or rate fading mode). The destination rate for the transition is called the "target rate" (see input "Target"). Each step towards the target is at a "next rate" (see output "Next") and steps occur at each cardiac cycle, as determined by the MTR. The next rate is fed into the rate multiplexer 302 at the "Rate Limiter" input.

In one embodiment the rate limiter component 305 works in intervals rather than rates. The attack interval change value and the decrement interval change value are rate dependent. The maximum delta (either up or down) for the next cardiac cycle interval is calculated by the processing unit 120 based on the current (selected) rate (interval) received from the rate multiplexer 302 (see input "Curr in"). The arithmetic required for this calculation is explained above.

The rate limiter component's 305 current interval typically comes from the interval it came up with as the next interval during the previous cycle (see feedback path), but when being initialized it is preloaded with the "prevailing rate", which is the average intrinsic VV delay. The current interval value is compared to the target interval (determined from target rate at the input) to see if it needs to speed up or slow down the rate in order to move towards the target interval at the maximum rate of change allowed. The next interval output is the largest step allowed in one cycle from the previous rate.

If usage of the subsequent rate fading mode is enabled (see input "RF Enable"), the current interval of the rate limiter component 305 is preloaded with (i.e. tracks) the average intrinsic VV delay whenever repetitive hysteresis is active, i.e. whenever ventricular senses have been detected recently.

The rate limiter target select component 307 selects the target rate for the rate limiter component 305 as described above being either a target based on the actual motion signal (see input motion signal) or one of the lower rates (basic rate, resting rate). This is selected based on the select signal sent by the control logic component 301.

The main timer component 310 is used to time each cardiac cycle. There will be a ventricular event (i.e. an intrinsic ventricular signal or ventricular pacing) in every cardiac cycle, but due to the far-field sensing approach for monitoring the intrinsic atrial signals, there may be cardiac cycles in which there is no detected intrinsic atrial signal, and thus the main timer component 310 is always synchronized by ventricular events. This timer component will be restarted on every used ventricular event (MTR) by the timer reset component 334 and it will always be running. The main timer component 310 is fed with the clock rate (128 Hz) as the AV timer component 312 and the hysteresis timer component 314, also called hysteresis component 314 or timer component 314. These two timer components 312, 314 are restarted through the MTR signal, as well.

The main timer component 310 may be used to determine the period within the cardiac cycle where it should be possible to separate intrinsic atrial signals from intrinsic ventricular signals. This time window is known as the atrial sense window. The atrial sense window will always be closed at every main timer reset.

There is a maximum rate at which ventricular paces should track intrinsic atrial signals. This rate may be a patient specific user programmable value which is called the upper tracking rate (UTR) by the programmer. The programmer converts this rate to an interval (UTI) for programming the pacemaker. When the corresponding time has been reached, a signal is sent to the pace component 320 to enable pacing (see input "UTI timeout").

In all pacing modes that are supported there is a maximum time interval starting from one ventricular event by which the pacemaker must provide a ventricular pace at the latest if no ventricular sense is detected. The interval corresponding with the programmed basic rate is timed by the main timer component 310 ("Main Timeout"). When this interval is exceeded before the timer is restarted, a ventricular pace will be triggered, because the main timeout is provided to the hysteresis timer component 314. It is further provided to the pace component 320 ("basic rate timeout"). This then is the minimum pacing rate. Because reaching this interval will always result in triggering a ventricular pace, which will in turn create a main timer reset, the main timer component 310 will never count past this value.

The processing unit 120 further comprises the AV timer component 312. It is used to measure AV delays and is started on every intrinsic atrial signal, wherein it may be restarted if a larger qualified intrinsic atrial signal is received by the processing unit 120. It is stopped and the counter is reset to 0 when a main timer reset (MTR) occurs.

The processing unit 120 further comprises a hysteresis logic component 330, also called hysteresis component 330, which is used only if hysteresis is enabled. As indicated above hysteresis is used to encourage intrinsic atrial or ventricular signals rather than pacing. For example, a second hysteresis delay is used to extend the AV delay to encourage ventricular sensing. Further, in the R-synchronized version of VDD where the current VV delay is used to time the ventricular pacing, if necessary, there are no atrial paces and a first hysteresis delay is applied to the VV delay in order to encourage ventricular sensing. Additionally, in order to cope with a sudden drop of the intrinsic cardiac rate (see explained below), the first hysteresis delay is used as described above. For this, the hysteresis logic component 330 comprises a counter. The hysteresis counter is set to a pre-defined number (e.g. a value between 10 and 3). In every cycle without any intrinsic ventricular signal the counter is decremented by 1. The counter is reset to the pre-defined number if an intrinsic ventricular signal is received by the processing unit. If the counter is decremented to zero, usage of hysteresis is stopped and the processing unit switches to the rate fading mode using the rate limiter target select component 307, the rate limiter component 305 and the rate multiplexer component 302 switches the rate input (from the "prevail" input to the "rate limiter" input). The information, whether hysteresis is active or not is sent to the hysteresis timer component 314.

The hysteresis timer component 314 is used to provide the first hysteresis delay and the second hysteresis delay as alternatives. The first hysteresis delay (which is a hysteresis interval) provides extra time before delivering a ventricular pace to encourage ventricular sense detection. The inputs to this timer include event triggers from the main timer component 310 reaching its time constant value and the AV timer component 312 reaching its time constant value. The second hysteresis delay is only provided if an intrinsic atrial signal is detected which information is received via the "As" input. If either the first hysteresis delay or the second hysteresis delay are timed out a signal encouraging the pacing signal (output "Do Vp") is provided to the pace component 320.

The processing unit 120 further comprises an interval monitor component 332 which measures intrinsic VV intervals (VV delays). The VV interval, in timer units, is captured in a register from the main timer value of the main timer component 310 just before it is reset by an MTR. This, along with event information from the cycle is used as the basis for the interval information provided by this component. For example, the interval monitor component may calculate an average value (e.g. an arithmetic average or a median value) using the last 5 to 10 measured VV intervals. The determined average value ("Average intrinsic" also referred to as current VV delay) is fed to the rate limiter component 305 and the rate multiplexer component 302 as input.

The VV interval starts with an MTR, e.g. an intrinsic ventricular signal or a ventricular pacing signal and ends with an intrinsic ventricular signal or a ventricular pacing signal.

In one embodiment VV intervals may be averaged using recursive filters and may be qualified before use for this average calculation depending on whether intrinsic signals were detected in this or previous cycles. If this cycle had an intrinsic atrial signal or ended in an intrinsic ventricular signal, then it was an intrinsic cycle. A cycle with an intrinsic atrial signal provides phase information, but not necessarily rate information. Two intrinsic cycles in a row provides rate information also and thereby forms an intrinsic interval. The time period between two MTR is called cardiac interval.

The pace component 320 of the processing unit triggers a ventricular pace, i.e. sends a ventricular pacing control signal (Vp) to the pacing signal generator 124. This control signal is based on the "Do Vp" signal of the hysteresis timer component 314 or the "Basic Rate Timeout" signal of the main timer component 310. The pace component 320 further needs to know whether the processing unit 120 is in the VDD mode. If it is and if the UTI (see above) has not yet timed out, the pace is delayed until the UTI timeout signal is received (as a minimum pacing interval corresponding to the UTR). If it is not in the VDD mode, the motion signal can trigger paces above the UTR up to the programmed maximum sensor rate (MSR limit). A motion sensor rate generator may limit the motion signal adapted VV delay by the MSR limit. Further, the pace component ensures a minimum interval from a detected intrinsic atrial signal using a signal from the AV timer component 312 (see input "Mn AV Timeout"). The pacing signal generator 124 uses the pacing time information contained in the ventricular pacing control signal (Vp) and produces the pacing signal based on further parameters such a pulse width, autoshort and blanking timing. The pacing signal generator 124 then transmits the pace signal to the electrodes 111, 112.

The processing unit 120 further comprises a timer reset component 334. Either a used intrinsic ventricular signal or a ventricular pace will trigger a main timer reset (MTR). The MTR resets the timer components 310, 312 and 314 and stops the timers of the first and second hysteresis delay. It provides the basis for cardiac cycle counting and clears logic that starts over for each cycle. The main timer value of the main timer component 310 is captured before the timers are reset and transmitted to the interval monitor component 332, providing a measurement of the actual VV delay and to calculate the current (average) VV delay using the actual VV delay if it is qualified.

In the following the behavior of the processing unit 120 is explained if in the VDD mode or the sensor mode the intrinsic cardiac rate drops sharply (i.e. the VV interval becomes significantly longer).

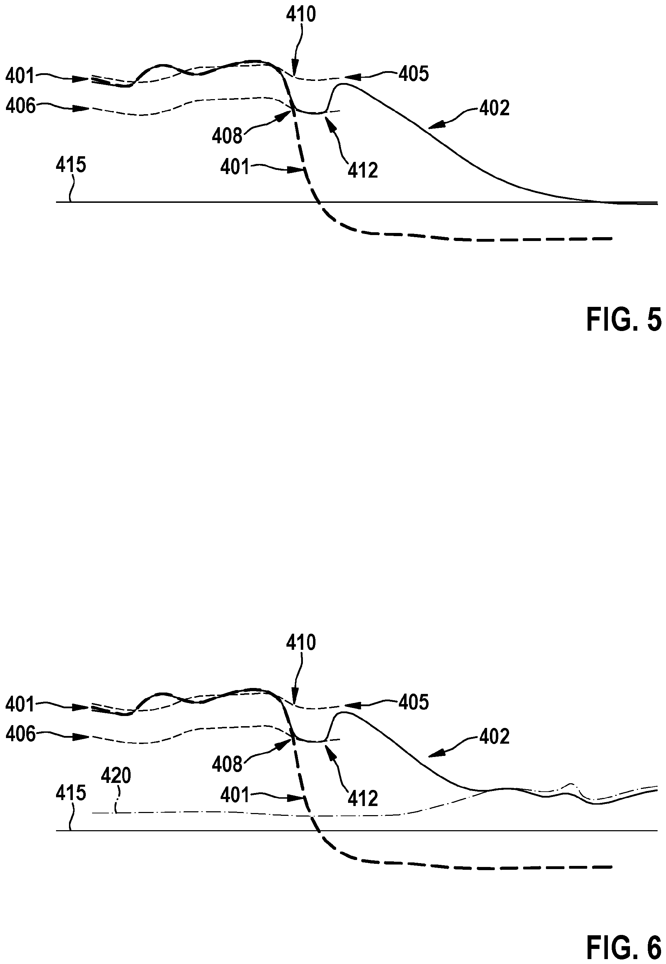

Fig. 5 shows the behavior of the processing unit in the VDD mode (without atrial tracking). The continuous line 401 represents the intrinsic cardiac rate determined by the processing unit. If the intrinsic cardiac rate drops significantly the pacing starts and the continuous line 401 branches out (see point 408) into two continuous lines, wherein the upper continuous line 402 represents the pacing rate and the lower continuous line 401 is the intrinsic cardiac rate. The upper dashed line 405 is the average intrinsic rate (corresponds to the average VV interval). The lower dashed line 406 is the average intrinsic rate minus the hysteresis rate (corresponds to the average current VV delay plus first hysteresis delay).

When the intrinsic cardiac rate drops, pacing picks up at the hysteresis delta below the last current VV delay (see reference number 408, where the continuous line branches out), wherein the last current VV delay is represented by value 410 in Fig. 5. After a pre-defined number of consecutive pacing cycles (e.g. between 3 and 10) without any intrinsic ventricular signal (counted by the counter of hysteresis logic component 330) the hysteresis delta is no longer applied (see point 412 in Fig. 3) because now the processing unit 120 switches to the rate fading mode and slowly decreases the rate (corresponding to the last current VV delay) to the basic rate (see line 415). At the beginning, the pacing rate jumps up a little because the initial rate value for rate fading is the rate corresponding to the last average VV delay (without hysteresis). This means that when hysteresis is no longer active, the target rate of the rate limiter component 305 is set to the basic rate and the pacing rate is ramped down at the allowed decrement rate change value (see line 402 in Fig. 3) using the rate limiter component 305. Accordingly, the rate multiplexer component 302 selects the rate received from the rate limiter component 305. This means that the VV delay for pacing corresponds to the rate provided by the rate limiter component 305. In course of time, the pacing rate approaches the basic rate (represented by line 415) as one can derive from Fig. 5.

Fig. 6 shows the same situation as for Fig. 5 but for the sensor mode. The behavior is similar to the one explained with respect to Fig. 5. However, in the sensor mode, there is additionally the sensor rate 420 which is the rate that corresponds to the detected motion signal of the motion sensor indicating the level of activity of the patient. The sensor rate is depicted in Fig. 6 with line 420. In the rate fading section starting at point 412 the rate fading selects the sensor rate as the target rate because it is enabled to use the signal of the motion sensor. Accordingly, the sensor rate is selected by the rate limiter target select component 307 (instead of the basic rate) and is provided at the target input of the rate limiter component 305. Hence, the pacing rate approaches the sensor rate during the course of time as can be seen in the rightmost section of line 402 in Fig. 6 representing the pacing rate.

The above explained embodiment of a cardiac pacemaker (ILP 10) and respective operation method realizes integrated circuit space conservation and simple design that covers many modes of operation even though robust behavior requires complex dynamic adaptive algorithms. The above design and operation provides an infrastructure that satisfies these needs.

## Claims

1. A cardiac pacemaker (10) for a patient's heart (20), for example an ILP, comprising a processing unit (120), a detector (126) and a pacing signal generator (124), wherein the processing unit, the detector and the pacing signal generator are electrically interconnected, wherein the detector is configured to detect electrical signals of the heart, for example an intracardiac electrogram (IEGM), and to transmit these signals to the processing unit (120), wherein the processing unit is configured to perceive an intrinsic ventricular signal and an intrinsic atrial signal from the signals received from the detector, to enable or disable the perception of the intrinsic atrial signal, to produce a ventricular pacing control signal (Vp) comprising a ventricular pacing time information and to transmit the pacing control signal to the pacing signal generator for providing a pacing signal for the patient's heart, wherein the processing unit is configured to produce the ventricular pacing control signal using a VDD mode or using at least one supplementary mode, wherein the processing unit is configured to conditionally use
- the VDD mode in the current cardiac cycle, if perception of the intrinsic atrial signal is enabled, wherein pacing in the VDD mode is based on a current AV delay if an intrinsic atrial signal is perceived by the processing unit within the current cardiac cycle or based on a current VV delay determined from previous intrinsic ventricular and/or atrial signals if an intrinsic atrial signal is not perceived by the processing unit within the current cardiac cycle and
- alternatively, the at least one supplementary mode in the current cardiac cycle based on the current VV delay which is determined from a previous VV delay considering at least one additional parameter or from a different assignment rule.

2. The pacemaker of claim 1, wherein a first supplementary mode is a VVI mode and a second supplementary mode is a sensor mode, wherein in the VVI mode the processing unit (120) is configured to determine the current VV delay from a basic rate, wherein in the sensor mode the processing unit is configured to determine the current VV delay based on signals of a sensor of the detector (126) detecting signals different from the intrinsic ventricular signals and the intrinsic atrial signals, for example motion signals, if perception of corresponding sensor signals is enabled.

3. The pacemaker of any of the previous claims, wherein, if in the VDD mode or the second supplementary mode an intrinsic ventricular signal is not perceived within the time interval of the current VV delay, the current VV delay is prolonged by a first hysteresis delay, wherein this prolongation is provided over a pre-defined number of consecutive cardiac cycles without any perceived intrinsic ventricular signal.

4. The pacemaker of claim 3, wherein the processing unit (120) is configured such that it uses a first subsequent mode, namely the rate fading mode, starting from the next cardiac cycle following the pre-defined number of consecutive cardiac cycles in which the prolongation of the current VV delay is provided by the first hysteresis delay and no intrinsic ventricular signal is perceived in the VDD mode or in the second supplementary mode, wherein in the rate fading mode the current VV delay is ramped up from a current VV delay to a VV delay corresponding to the basic rate or a current sensor rate.

5. The pacemaker of any of the previous claims, wherein the processing unit (120) is configured to switch into a second subsequent mode, namely a FindSync mode, in which the processing unit seeks to perceive intrinsic atrial signals and atrial tracking opportunities if in the first supplementary mode, the rate fading mode or in the sensor mode the current VV delay corresponds to the basic rate or a resting rate for a pre-defined time interval.

6. The pacemaker of any of the previous claims, wherein the processing unit (120) comprises a rate limiter component (305) which determines the current rate for ramping the VV delay in the rate fading mode or for adaption of the current VV delay to the detected sensor signals in the sensor mode, wherein rate limiter component provides an attack rate change value and a decrement rate change value.

7. The pacemaker of any of the previous claims, wherein the processing unit (120) comprises a hysteresis component (330, 314) which provides the first hysteresis delay for the VDD mode and the second supplementary mode and preferably also a second hysteresis delay for the VDD mode for prolongation of the AV delay.

8. An operation method of a cardiac pacemaker (10) for a patient's heart (20), for example an ILP, comprising a processing unit (120), a detector (126) and a pacing signal generator (124), wherein the processing unit, the detector and the pacing signal generator are electrically interconnected, wherein electrical signals of the heart, for example an intracardiac electrogram (IEGM), are detected by the detector and transmitted to the processing unit (120) for perception of an intrinsic ventricular signal and an intrinsic atrial signal from the signals received from the detector, wherein the perception of the intrinsic atrial signal can be enabled or disabled by the processing unit, wherein a ventricular pacing control signal (Vp) comprising a ventricular pacing time information is produced by the processing unit and transmitted to the pacing signal generator for pacing signal generation for the patient's heart, wherein the ventricular pacing control signal is produced conditionally using a VDD mode or at least one supplementary mode, wherein
- the VDD mode is used in the current cardiac cycle, if perception of the intrinsic atrial signal is enabled, wherein pacing in the VDD mode is based on a current AV delay if an intrinsic atrial signal is perceived by the processing unit within the current cardiac cycle or based on a current VV delay determined from previous intrinsic ventricular and/or atrial signals if an intrinsic atrial signal is not perceived by the processing unit within the current cardiac cycle and
- alternatively, the at least one supplementary mode is used in the current cardiac cycle based on the current VV delay which is determined from a previous VV delay considering at least one additional parameter or from a different assignment rule.

9. The method of claim 8, wherein a first supplementary mode is a VVI mode and a second supplementary mode is a sensor mode, wherein in the VVI mode the current VV delay is determined from a basic rate, wherein in the sensor mode the current VV delay is based on signals of a sensor of the detector (126) detecting signals different from the intrinsic ventricular signals and the intrinsic atrial signals, for example motion signals, if perception of corresponding sensor signals is enabled. [in the sensor mode perception of atrial signals is disabled, the sensor is configured to detect the corresponding signals]

10. The method of any of the claims 8 to 9, wherein, if in the VDD mode or the second supplementary mode an intrinsic ventricular signal is not perceived within the time interval of the current VV delay, the current VV delay is prolonged by a first hysteresis delay, wherein this prolongation is provided over a pre-defined number of consecutive cardiac cycles without any perceived intrinsic ventricular signal, wherein additionally or alternatively a second hysteresis delay for prolongation of the AV delay is used in the VDD mode, wherein the first hysteresis delay and/or the second hysteresis delay may be provided, for example, by a hysteresis component (330, 314) of the processing unit.

11. The method of claim 10, wherein a first subsequent mode is used, namely a rate fading mode, starting from the next cardiac cycle following the pre-defined number of consecutive cardiac cycles in which the prolongation of the VV delay is provided by the first hysteresis delay and no intrinsic ventricular signal is perceived in the VDD mode or in the second supplementary mode, wherein in the rate fading mode the VV delay is ramped up from a current VV delay to a VV delay corresponding to a basic rate or a current sensor rate.

12. The method of any of the claims 8 to 11, wherein the method will switch into a second subsequent mode, namely a FindSync mode, in which the processing unit (120) seeks to perceive intrinsic atrial signals and atrial tracking opportunities if in the rate fading mode or in the sensor mode the current VV delay corresponds to the basic rate or a resting rate for a pre-defined time interval.

13. The method of any of the claims 8 to 12, wherein the current rate for ramping the VV delay is determined in the rate fading mode or for adaption of the current VV delay to the detected sensor signals in the sensor mode by a rate limiter component (305), wherein the rate limiter component provides an attack rate change value and a decrement rate change value.

14. A computer program product comprising instructions which, when executed by a processing unit, cause the processing unit (120) to perform the steps of the method according to any of the claims 8 to 13.

15. Computer readable data carrier storing a computer program product according to claim 14.
